(19) Europäisches Patentamt
European Patent Office
Office européen des brevets

(11) Publication number: 0 219 838 A2

(12) # EUROPEAN PATENT APPLICATION

(21) Application number: 86114466.5

(22) Date of filing: 18.10.86

(51) Int. Cl.[4]: C07D 473/30 , C07D 473/18 , C07D 233/90 , C07D 405/04 , C07F 7/08 , A61K 31/52

A request for correction of the text has been filed pursuant to Rule 88 EPC. A decision on the request will be taken during the proceedings before the Examining Division (Guidelines for Examination in the EPO, A-V, 2.2).

(30) Priority: 22.10.85 JP 236858/85
22.10.85 JP 236859/85
13.08.86 JP 190830/86

(43) Date of publication of application: 29.04.87 Bulletin 87/18

(84) Designated Contracting States: AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Taniyama, Yoshio
6-31, Zuiko 1-chome
Higashiyodogawa-ku Osaka 533(JP)
Inventor: Marumoto, Ryuji
53-1 Okuikeminami-machi
Ashiya Hyogo 659(JP)

(74) Representative: von Kreisler, Alek, Dipl.-Chem.
Deichmannhaus am Hauptbahnhof
D-5000 Köln 1(DE)

(54) ## Carbocyclic purine nucleosides, their production and use.

(57) The cárbocyclic analogs to purine nucleosides represented by the formula

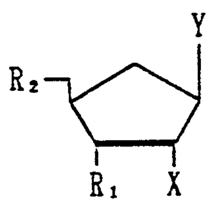

wherein $R_1$ and $R_2$ each is a hydroxyl group, which may optionally be protected, X is a hydrogen atom or a hydroxyl group, which may optionally be protected, and Y is guanin-9-yl, isoguanin-9-yl or hypoxanthin-9-yl are useful for the treatment of viral infections and as substitutes for purine nucleosides in the field of genetic engineering, biochemistry or the like.

EP 0 219 838 A2

# Carbocyclic Purine Nucleosides, Their Production and Use

This invention provides carbocyclic analogs to purine nucleosides which are usable as substitutes for purine nucleosides in the field of genetic engineering, biochemistry or the like and as an antiviral agent.

As examples of carbocyclic analogs to purine nucleosides, there are known aristeromycin of the formula

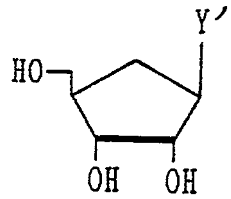

wherein Y' is adenin-9-yl [Chemical Communications, 852 (1967)] and a compound of the above formula wherein Y' is hypoxanthin-9-yl [Chemical & Pharmaceutical Bulletin, 24, 2624 (1976)]. The above compounds are carbocyclic analogs - (hereinafter sometimes abbreviated to "C-analogs") to adenosine and inosine, respectively.

It has already been established that cyclopentanediol type purine nucleoside analogs, typically aristeromysin, can serve effectively as substrates in various enzyme systems in which ordinary purine nucleosides are involved [Journal of Biochemistry, 73, 945 (1973)].

Furthermore, C-analogs to purine nucleosides have recently attracted attention as antiviral agents [Science, 227, 1296 (1985)]. On the other hand, DNAs containing C-analogs to 2'-deoxypurine nucleotides have some characteristics which can advantageously be utilized in gene manipulation as compared with DNAs containing deoxyadenosine - [Japanese Patent Applications Nos. 73822/1984 and 258789/1984].

However, C-analogs to deoxyguanosine and C-analogs to guanosine remain uninvestigated in many fields. It is an important task to synthesize and evaluate various further C-analogs.

Under these circumstances, the present inventors conducted investigations in order to obtain novel and useful C-analogs to purine nucleosides and have now completed the present invention.

Thus, the invention provides:

(1) A compound of the formula [I]

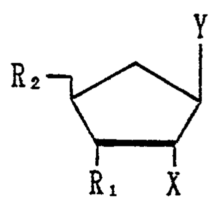

[I]

wherein $R_1$ and $R_2$ each independently is a hydroxyl group, which may optionally be protected, X is a hydrogen atom or a hydroxyl group, which may optionally be protected, and Y is guanin-9-yl, isoguanin-9-yl or hypoxanthin-9-yl, provided that when X is a hydroxyl group, which may optionally be protected, Y is other than hypoxanthin-9-yl;

(2) A method of producing the compounds of the formula [I] which comprises subjecting a compound of the formula [II]

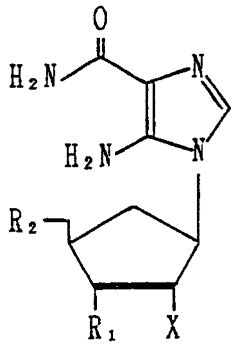

[II]

wherein $R_1$ and $R_2$ are each independently is a hydroxyl group, which may optionally be protected, and X is a hydrogen atom or a hydroxyl group, which may optionally be protected, to purine ring formation reaction; and

(3) An antiviral composition containing a compound of the formula [I']

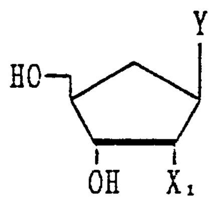

[I']

wherein $X_1$ is a hydrogen atom or a hydroxyl group, and Y is guanin-9-yl, isoguanin-9-yl, or hypoxanthin-9-yl, provided that when $X_1$ is a hydroxyl group, Y is other than hypoxanthin-9-yl.

Referring to the compounds of the formula [I] or [II], when $R_1$, $R_2$ and/or X is a protected hydroxyl group, the protective group should preferably be stable under alkaline conditions. Thus, for example, there may be mentioned alkylsilyl groups having 3-10 carbon atoms (e.g. dimethyl-t-butylsilyl), alkyl or alkoxy cyclis ethers having 4-10 carbon atoms (e.g. tetrahydrofuranyl and its alkyl derivatives having 4-7 carbon atoms, tetrahydropyranyl and its alkyl derivatives having 5-8 carbon atoms such as methoxytetrahydropyranyl), alkoxy-alkyl groups having 3-10 carbon atoms (e.g. ethoxymethyl, methoxyethyl), trityl and alkoxy substituted trityl groups (e.g. monomethoxytrityl, dimethoxytrityl) .

The compounds of the formula [I] are obtained by subjecting the compounds of the formula [II] to purine ring formation reaction. The purine ring formation can be effected by using those reaction methods that are known for that purpose in heterocyclic chemistry. For example, the compounds of general formula [I] wherein Y is hypoxanthin-9-yl can be produced in the manner mentioned below. Thus, the compounds of general formula [II] are reacted with a variety of esters (e.g. esters between straight or branched aliphatic carboxylic acids having 2-20 carbon atoms or aromatic carboxylic acids having 7-30 carbon atoms and alcohols having 1-10 carbon atoms) with heating in the presence of a sodium alcoholate (e.g. ethylate, methylate, tert-butylate) to give 2-alkyl-or 2-aryl-substituted C-analogs to inosine or 2'-deoxyinosine. When heated with formamide or formic acid, they give C-analogs to inosine or 2'-deoxyinosine, respectively. It is also possible to first produce C-analogs to 2-mercaptoinosine or 2'-dexoy-2-mercaptoinosine by heating the compounds [II] with phenyl isothiocyanate or potassium xanthate, respectively, in pyridine and then derive therefrom 2-alkoxy (e.g. methoxy, ethoxy, butoxy) or 2-substituted-amino (e.g. methylamino, dimethylamino, ethylamino, diethylamino, cyclohexylamino, morpholino) derivatives via the corresponding 2-halo (e.g. chloro, bromo) intermediates. For the compounds of the formula [I] in which Y is guanin-9-yl, the process involving the above-mentioned C-analogs to 2-mercaptoinosine or 2'-deoxy-2-mercaptoinosine as intermediates [Chem. Pharm. Bull., 19 , 576 (1971)] can be used. Furthermore, in accordance with the description in Nucleic Acids Res., 3, 251 (1976), the process which comprises, for example, reacting a compound of the formula - [II] with benzoyl isothiocyanate in boiling acetone, methylating the resulting benzoylthiourea derivative with methyl iodide in an acetone-potassium carbonate mixture or with dimethyl sulfate in an aqueous sodium hydroxide solution and further heating

the methylation product in about 2-6 N sodium hydroxide to thereby cause ring closure may also be used.

On the other hand, the benzoylmethylisothiourea intermediate obtained in the above-mentioned process, when heated for ring closure in a relatively low concentration alkali solution, such as about 0.1-1 N sodium hydroxide, gives a compound of the formula [I] in which Y is isoguanin-9-yl.

The trityl and other ether-forming hydroxyl-protecting groups in the compounds of the formula [I] can be eliminated easily by a conventional method using various organic acids (e.g. acetic acid, trichloroacetic acid, dichloroacetic acid, benzenesulfonic acid, toluenesulfonic acid) or various inorganic acids [e.g. hydrochloric acid, sulfuric acid, various Lewis acids (zinc bromide, aluminum chloride, etc.)]. The hydroxyl-protecting silyl groups may be eliminated with acids such as mentioned above but can be removed under milder conditions by using tetrabutylammonium fluoride.

The compounds of the formula [II] to be used in the production method according to the invention can be obtained by treating a compound of the formula [III]

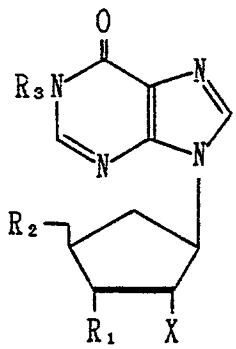

[III]

wherein $R_1$, $R_2$ and X are as defined above and $R_3$ is an alkoxymethyl group, with an alkali.

The alkoxymethyl group represented by $R_3$ generally contains 2-7 carbon atoms and includes among others methoxymethyl, ethoxymethyl, propoxymethyl and phenoxymethyl. The methoxymethyl group is particularly useful among them.

For the alkali treatment mentioned above, an alkali hydroxide can preferably be used. Thus, for example, the treatment can be conducted by heating at a temperature within the range of about 60°-200°C in about 1-5 M aqueous sodium hydroxide or potassium hydroxide solution or in a mixture of such alkali solution and a water-soluble neutral organic solvent (e.g. alcohol, dioxane). In this case, the reaction time is generally about 10 minutes to about 2 hours.

The compounds of formula [III] to be used for the above reaction are prepared by converting the adenine ring of aristeromycin to the hypoxanthine ring [Chem. Pharm. Bull., 24, 2624 (1976)], then introducing, a hydroxyl-protecting group or groups represented by $R_1$, $R_2$ or/and X as necessary and further introducing an alkoxymethyl group represented by $R_3$.

The introduction of hydroxyl-protecting group can be carried out by a per se known method [e.g. Can. J. Chem., 60 111 (1982)]. In case the compounds [III] have such protective group or groups, the reaction products after ring cleavage of the hypoxanthine ring by the above-mentioned alkali treatment can be purified with convenience. This protective group introduction is carried out, for example by reaction with the alkylsilyl chlorides, the alkoxy alkyl chlorides or the trityl and alkoxy substituted trityl chlorides, or the alkyl or alkoxy cyclic ethers in an organic solvent such as pyridine, dimethylformamide, dichloromethane, chloroform, dioxane, ethyl ether, acetonitrile, dimethyl sulfoxide or nitromethane.

The compounds of formula [III] in which X is hydrogen can be obtained by protecting the hydroxyl group $R_1$ and/or $R_2$ in advance, converting the hydroxyl group at the position of X to the thiocarbonylated forms (e.g. phenylthioformate ester form, thiocarbonyl imidazolide form) and then converting the same to the 2'-deoxy from in the manner of radical reaction using an organotin hydride. For this purpose, the procedure described in J. Org. Chem., 47, 485 (1982) can be used.

The introduction of an alkoxymethyl group represented by $R_3$ can be carried out using the method described in J. Am. Chem. Soc., 24, 2624 - (1976), for instance. This alkoxymethyl group introduction advantageously enables performance of the above-mentioned hypoxanthine ring cleavage under relatively mild conditions and further enables easy elimination of said group in a later treatment step as compared with the case where $R_3$ is a

simple alkyl group. In the above manner, there can be obtained the compounds of formula [III].

The compounds of formula [I] according to the invention can be used in the field of genetic engineering or biochemistry as equivalents to nucleosides containing guanine, isoguanine or hypoxanthine as the base. Thus, for instance, the compounds of formula [I] in which Y is guanin-9-yl can be used in lieu of dGTP which serves as a substrate for DNA polymerase. DNAs thus obtained and containing such carbocyclic analogs to guanosine are useful in various areas of recombinant DNA technology.

On the other hand, the compounds of formula - [I'] according to the present invention have potent antiviral activity against DNA virus. Examples of such virus include herpes virus group (e.g. herpes simples virus type I or II, varicella-zoster virus), adenovirus (e.g. type 3) or vacciniavirus. The compounds can be used as antiviral agent for the treatment of virus-induced disease in animals, particularly in mamalian animals (laboratory animals such as rabbit, rat and mouse; pet animals such as dog and cat; and human being).

For use in the treatment mentioned above, the compounds of the present invention can be orally or otherwise administered, either as it is or in combination with an appropriate pharmacologically acceptable carrier, excipient or diluent, in such dosage forms as powders, granules, tablets, capsules, solutions, ointments and injections. There may be mentioned as carrier lactose, starch, mineral oil, petroleum jelly, polyethyleneglycol, propyleneglycol, saline solution for injection on the like. The dosage depends on the kind of virus, symptoms, administration hosts and routs or the like. For the treatment of herpes virus-induced disease in an adult human being, for instance, the compound is desirably administered in a vein at a daily dose level of about 1 to 10 mg in a single dose or up to 3 divided portions. For the oral route, the compound is desirably administered at a level of about 10 to 100 mg per a dose in a single or up to 3 divided portions.

The following reference examples, working examples and test examples are further illustrative of the invention.

Example 1

Synthesis of 9-[(1R, 2S, 3R, 4R)-4-methyl-2-hydroxyl-3,6-(tetraisopropyldisiloxanyl)-dioxycyclopentan-1-yl]-hypoxanthine.

The C-analog to inosine [i.e. compound of general formula (I) in which $R_1$, $R_2$ and x each is a hydroxyl group and Y is hypoxanthin-9-yl] (10 g, 37.5 mmol) was dissolved in 200 ml of anhydrous DMF, 1,3-dichloro-1,1,3,3-tetraisopropyldisiloxane - (13 ml, 41 mmol) and imidazole (11.3 g, 165 mmol) were added, and the mixture was stirred at room temperature for 2.5 hours. The reaction mixture was added dropwise to 2 liters of water, and the precipitate was collected by filtration, washed with water, further washed quickly with diethyl ether and dried to give the title compound as a white powder (17.2 g). A portion of the product was recrystallized from dichloromethane to give crystals, m.p. 135-138°C.

Example 2

Synthesis of 9-[(1R, 2S, 3R, 4R)-4-methyl-2-phenoxy-thiocarbonyloxy-3,6-(tetraisopropyldisiloxanyl)-dioxycyclopentan-1-yl]-hypoxantine

The compound obtained in Example 1 (11.2 g, 22.3 mmol) was dissolved in 300 ml of anhydrous acetonitrile, dimethylaminopyridine (15.8 g, 53.3 mmol) and phenoxythiocarbonyl chloride (5 g, 29 mmol) were added and the mixture was stirred at room temperature for 7 hours. The solvent was removed under reduced pressure, and the residue was dissolved in 250 ml of chloroform. The solution was washed with 0.5 M potassium dihydrogen phosphate solution (250 ml x 2) and then with water (200 ml), dried (anhydrous sodium sulfate) and concentrated under reduced pressure to give a yellow syrup. This was purified by silica gel chromatography (90 g; solvent: $CHCl_3$ and $CHCl_3$/MeOH -60/1) to give the title compound as a light-yellow grass-like substrate (13.0 g).

NMR(60MHz, $CDCl_3$ $\delta$ ppm: 1.0-1.23 (28H, m), 2.13-2.43 (3H, m, H4' H5'), 3.93-4.10 (2H, m, $H_6$'), 4.80-5.20 (2H, m, $H_1$', $H_3$'), 6.00-6.20 (H, m, $H_2$'), 7.03-7.50 (5H, m), 7.87 (1H, s), 8.13 (1H, s) s;singlet, m;multiplet

Example 3

Synthesis of 9-[(1R, 3R, 4R)-4-methyl-3,6-(tetraisopropyldisiloxanyl)dioxycyclopentan-1-yl]-hypoxanthine

Anhydrous toluene (30 ml) was added to the compound obtained in Example 2 (13.0 g, 20 mmol), followed by concentration under reduced pressure. The residue was dissolved in 300 ml of anhydrous toluene, and nitrogen gas was bubbled into the solution for 20 minutes. After addition of tributyltin hydride (11 ml, 40 mmol), the solution was heated at 80°C and crystal line $\alpha$, $\alpha$'-azobisisobutyronitrile (AIBN) (820 mg) was added in 4 portions at 15-minute intervals. After 3 hours of

heating with stirring, the solvent was removed under reduced pressure. The oil obtained was purified by silica gel chromatography (80 g; solvent: $CHCl_3$ and $CHCl_3$/MeOH = 60/1 to 30/1) to give the title compound as a colorless glass-like substance - (10.4 g). Recrystallization of a portion of the product from ethanol gave colorless needles, m.p. 200-202°C.

NMR(60MHz, $CDCl_3$)δ ppm: 0.93-1.20 (28H, s), 1.97-2.53 (5H, m, $H_2$', $H_4$', $H_5$'), 3.80-4.07 (2H, m, $H_6$'), 4.43-5.27 (2H, m, $H_1$', $H_3$'), 7.87 (1H, s), 8.20 - (1H, s)

Example 4

Synthesis of 9-[(1R, 3R, 4R)-4-(monomethoxytrityloxy)-methyl-3-hydroxylcyclopentan-1-yl]-(1-methoxymethyl-hypoxantine)

The compound obtained in Example 3 (9.8 g, 19.8 mmol) was dissolved in 240 ml of anhydrous dioxane, sodium hydride (880 mg, 21.8 mmol) was quickly added to the solution with ice cooling and stirring and, then, the mixture was stirred at room temperature for 1.5 hours. Thereafter, methoxymethyl chloride (2 ml, 21.8 mmol) was quickly added to the mixture with ice cooling. The whole mixture was stirred at room temperature for 3 hours.

The solvent was removed under reduced pressure and the oily residue was dissolved in 200 ml of chloroform. The solution was washed with 0.1 M triethylammonium bicarbonate (TEAB) buffer (ph 7.5, 100 ml x 2) and further with water (200 ml), dried (anhydrous sodium sulfate) and concentrated under reduced pressure to give a syrup. Purification of the syrup by $C_{18}$ silica gel chromatography - (Ø 5.3 x 7.0 cm; solvent: acetone water, 55%-80%) gave a colorless glass-like compound (8.5 g).

This compound (8.0 g) was dissolved in 32 ml of tetrahydrofuran (THF), tetrabutylammonium fluoride trihydrate (TBAF•$3H_2O$) (10 g) was added, and the mixture was stirred at room temperature for 0.5 hour. The solvent was removed under reduced pressure and the remaining oil was dissolved in 100 ml of water. The solution was washed with diethyl ether (100 ml x 2) and was deprived of the tetrabutylammonium salt by treatment on Dowex-50 resin (pyridine form 60 ml). The effluent and water washings (240 ml) were combined and concentrated and the concentrate was dehydrated azeotropically with three portions of pyridine. The residue was dissolved in 100 ml of pyridine, monomethoxytrityl chloride (MMTrCl) (5.4 g) was added and the mixture was stirred at 37°C for 4 hours. The solvent was removed under reduced pressure and the oily residue was distributed between 0.1 M TEAB buffer (50 ml) and $CHCl_3$ (100 ml), the organic layer was washed with water (100 ml), dried - (anhydrous sodium sulfate) and concentrated under reduced pressure and the concentrate was subjected to dehydration by azeotropic distillation with toluene to give a colorless syrup. 0.1 M-TEAB buffer fraction and water washings were combined and concentrated, whereby the unmonomethoxytritylated compound was recovered. This compound was purified on HP-20 resin (190 ml; solvent: water and 30% ethanol-water) and, after concentration and azeotropic distillation with pyridine, monomethoxytritylated in the same manner as mentioned above. Both the thus-obtained crops of the title compound were combined and purified by silica gel chromatography (80 g; solvent: $CHCl_3$/MeOH = 100/1, 60/1, 50/1) to give a colorless glass-like product (6.1 g). A solution of a portion of this product in dichloromethane, when added dropwise to n-hexane, gave a white powder.

NMR(60 MHz, $CDCl_3$)δ ppm: 1.87-2.70 (5H, m, $H_2$', $H_4$', $H_5$'), 3.20-3.40 (2H, m, $H_6$'), 3.43 (3H, s, C<u>H</u>$_3$OCH$_2$), 3.80 (3H, s), 4.30-4.57 (1H, m, $H_3$'), 4.87-5.10 (1H, m, $H_1$'), 5.47 (2H, s, CH$_3$OC<u>H</u>$_2$-N), 6.73-6.97 (2H, m), 7.17-7.53 (12H, m), 7.73 (1H, s), 7.98 (1H, s)

Example 5

Synthesis of 1-[(1R, 3R, 4R) -4-(monomethoxytrityloxy)-methyl-3-hydroxycyclopentan-1-yl]-(4-carbamoyl-5-aminoimidazole)

The compound obtained in Example 4 (6.1 g, 10.7 mmol) was dissolved in 490 ml of ethanol and, with heating under reflux, a warmed 5 M aqueous sodium hydroxide solution (130 ml) was added quickly. Refluxing was continued for additional 40 minutes. The solvent was then removed under reduced pressure. The oily residue was dissolved in 200 ml of chloroform and washed with water (100 ml x 2), then with 0.1 M-TEAB buffer (100 ml x 2) and further with saturated aqueous sodium chloride solution (100 ml), dried (anhydrous sodium sulfate) and concentrated under reduced pressure to give a syrup. Purification of this syrup by silica gel chromatography (90 g; solvent: $CHCl_3$/MeOH = 100/1 to 20/1) gave a colorless glass-like product - (3.2 g). A solution of a portion of this product in chloroform, when added dropwise to n-pentane with stirring, gave a white powder.

Elemental analysis (%) for $C_{30}H_{32}N_4O_4$•0.5 $H_2O$, molecular weight 521.616

Calculated : C, 69.08; H, 6.38; N, 10.74

Found : C, 69.14; H, 6.09; N, 10.54

NMR (100MHz,$CDCl_3$) δ ppm: 1.36-2.52 (5H, m), 3.00-3.40 (3H, m, $H_6'$, OH), 3.77 (3H, s), 4.12-4.60 (2H, m, $H_1'$, $H_3'$), 4.80-5.28 (2H, bs, $NH_2$), 5.64-6.44 (2H, bs, $NH_2$), 6.76-6.94 (3H, m), 7.14-7.48 (12H, m) bs;broad singlet

Example 6

Synthesis of 1-[(1R, 3R, 4R)-4-hydroxymethyl-3-hydroxycyclopentan-1-yl]-(4-carbamoyl-5-aminoimidazole)

The compound obtained in Example 5 (2.3 g, 4.4 mmol) was dissolved in 50 ml of 80% acetic acid and the solution was stirred at 40°C for 7 hours. After concentration under reduced pressure, azeotropic distillation was conducted with toluene and then with ethanol. The residue was dissolved in 12 ml of ethanol. To the solution was added dropwise with stirring 130 ml of n-hexane-ether (1:1, v/v). The thus-obtained syrup was purified by $C_{18}$ silica gel chromatography (10 g; solvent: water and 50% acetone-water). The residue obtained after removal of the solvent under reduced pressure was recrystallized from ethanol to give the title compound (0.93 g), m.p. 162-163°C.

$\lambda_{max}$ (nm): ($H_2O$); 234 (sh), 268

($H^+$) ; 244, 269

($OH^-$); 267.5 sh; shoulder

NMR(60MHz,DMSO-$d_6$ + $D_2O$)δ ppm: 1.67-2.67 (5H, m), 3.43-3.60 (2H, m, $H_6'$), 3.90-5.00 (2H, m), 7.23 (1H, s, $H_2$); (DMSO-$d_6$) 5.77 (2H, bs, $NH_2$), 6.63 (2H, bs, $NH_2$)

Elemental analysis (%) for $C_{10}H_{16}N_4O_3$, molecular weight 240.262

Calculated : C, 49.99; H, 6.71; N, 23.32

Found : C, 49.32; H, 6.34; N, 22.92

Example 7

Synthesis of 1-[(1R, 3R, 4R)-4-(monomethoxytrityloxy)-methyl-3-hydroxycyclopentan-1-yl]-[4-carbamoyl-5-(N-benzoyl-S-methylisothiocarbamoyl) aminoimidazole]

The compound obtained in Example 5 (0.88 g, 1.7 mmol) was dissolved in 25 ml of anhydrous acetone and, with heating under reflux, a solution of benzoyl isothiocyanate (260µl, 1.9 mmol) in acetone (8 ml) was added dropwise over 10 minutes, followed by refluxing for 50 minutes. The solvent was removed under reduced pressure and the light-yellow glass-like substance obtained was purified by silica gel chromatography (15 g; solvent: $CHCl_3$/MeOH = 50/1 to 30/1) to give a light-yellow glass-like compound (0.87 g). A small amount of acetone was added to this compound (0.84 g, 1.2 mmol) and the resultant syrup was converted to a homogeneous solution by addition of 12.5 ml of 0.2 N NaOH and sonication. Dimethyl sulfate (130 µl, 1.4 mmol) was added with stirring and, then, vigorous stirring was continued at room temperature for 1 hour. The reaction mixture was mixed with $CHCl_3$ (15 ml x 2) for partition, the organic layer was washed with 0.1 M TEAB buffer (15 ml x 3) and then with saturated aqueous sodium chloride solution (20 ml), dried (anhydrous sodium sulfate) and concentrated under reduced pressure, and the residue was purified by silica gel chromatography (15 g; solvent: $CHCl_3$/MeOH = 100/1 to 60/1). A small amount of dichloromethane was added to the glass-like substance obtained, the mixture was added dropwise to hexane and the resultant precipitate was collected by centrifugation and dried to give the title compound as a powder (400 mg).

Elemental analysis (%) for $C_{39}H_{39}N_5O_5S$, molecular weight 689.835

Calculated : C, 67.90; H, 5.70; N, 10.15;

Found : C, 67.45; H, 5.45; N, 9.89;

NMR (100 MHz, $CDCl_3$) δ ppm: 1.34-2.60 (5H, m), 2.52 (3H, s, $SCH_3$), 3.04-3.44 (2H, m, $H_6'$), 3.79 (3H, s, $OCH_3$), 4.08-4.44 (1H, m, $H_3'$), 4.60-5.00 (1H, m, $H_1'$), 5.64 (1H, bs, $NH_2$), 6.72-6.94 (3H, m), 7.12-7.52 (15H, m), 7.80-7.96 (2H, m), 11.35 (1H, bs, NH)

Example 8

Synthesis of 1-[(1R, 3R, 4R)-4-hydroxymethyl-3-hydroxycyclopentan-1-yl]-[4-carbamoyl-5-(N-benzoyl-S-methylisothiocarbamoyl)-aminoimidazole]

The compound obtained in Example 6 (815 mg, 3.4 mmol) was reacted with 1.1 equivalents of benzoylisothiocyanate in acetone. The solvent was then removed under reduced pressure, 15 ml of acetone-$CHCl_3$ (2:1, v/v) was added and ether was

further added. The resultant precipitate was collected by filtration and dried. The thus-obtained powder (1.4 g) was dissolved in 35 ml of 0.2 N sodium hydroxide, dimethyl sulfate 340 µl) was added and the mixture was stirred at room temperature for 1 hour. Acetic acid was added to the reaction mixture with ice cooling (to pH 4-5), the product was extracted from the white, turbid reaction mixture with n-butanol (20 ml x 3), the extract was washed with water (10 ml x 2), the solvent was removed under reduced pressure and the residue was purified by silica gel chromatography (10 g; solvent: water and 30% acetone-water) to give a light-yellow glass-like substance (920 mg). Recrystallization from water gave the title compound as colorless crystals (570 mg), m.p. 119-120°C.

Elemental analysis (%) for $C_{19}H_{23}N_5SO_4 \bullet 0.3H_2O$, molecular weight 422.886

Calculated : C, 53.96; H, 5.62; N, 16.56; S, 7.58

Found : C, 54.03; H, 5.49; N, 16.44; S, 7.53

The following were confirmed: NMR (100 MHz, DMSO-$d_6$) δ ppm: 2.52 (3H, s, $CH_3$), 7.34-7.94 (6H, m), 11.85 (1H, bs, NH).

Example 9

Synthesis of 9-[(1R, 3R, 4R)-4-monomethoxytrityloxymethyl-3-hydroxycyclopentan-1-yl]guanine

The compound obtained in Example 7 (360 mg, 0.53 mmol) was added to a warmed 6 N sodium hydroxide (18 ml) and the mixture was heated under reflux for 1 hour. The product was extracted from the reaction mixture with $CHCl_3$, and the extract was washed with 0.1 M TEAB buffer (30 ml) and then with saturated aqueous sodium chloride solution (30 ml), dried (anhydrous sodium sulfate) and subjected to silica gel chromatography (8 g; solvent: $CHCl_3$/MeOH = 40/1 to 6/1). To the thus-obtained glass-like substance was added a small amount of acetone, the mixture was added dropwise to benzene and the resultant precipitate was collected by centrifugation and dried to give the title compound as a powder (210 mg).

Elemental analysis (%) for $C_{31}H_{31}N_5O_4 \bullet 1.0H_2O$, molecular weight 555.633

Calculated : C, 67.01; H, 5.99; N, 12.60

Found : C, 67.01; H, 5.69; N, 12.42

NMR (100MHz,DMSO-$d_6$) δ ppm: 1.50-2.60 (5H, m), 3.01 (2H, bs), 3.98-4.20 (1H, m), 4.70-4.96 (2H, m), 6.37 (2H, bs, $NH_2$), 6.82-7.46 (14H, m), 7.68 - (1H, s, $H_8$), 10.60 (1H, bs, NH)

Example 10

Synthesis of 9-[(1R, 3R, 4R)-4-hydroxymethyl-3-hydroxycyclopentan-1-yl]guanine

The compound obtained in Example 9 (180 mg, 0.33 mmol) was dissolved in 10 ml of 80% acetic acid and the solution was stirred at 40°C for 4.5 hours. The solvent was removed under reduced pressure and, further, azeotropic distillation was conducted twice with water. Water (10 ml) was added, the mixture was washed with ether (10 ml x 2) and the water was removed under reduced pressure. Thus was obtained the title compound as colorless crystals (41 mg), m.p. 246-248°C.

$[\alpha]_D^{25} = +7.7°$ (C=0.5, DMF)

$\lambda_{max}$ (nm): ($H_2O$); 255, 278 (sh)

($H^+$) ; 257, 282

($OH^-$); 256 (sh), 273

Elemental analysis (%) for $C_{11}H_{15}N_5O_3 \bullet 0.5H_2O \bullet 0.1C_2H_5OH$, molecular weight 278.886

Calculated: C, 48.24; H, 6.00; N, 25.11

Found : C, 48.61; H, 6.41; N, 25.40

Example 11

Synthesis of 9-[(1R, 3R, 4R)-4-monomethoxytrityloxymethyl-3-hydroxycyclopentan-1-yl]isoguanine

The compound obtained in Example 7 (585 mg, 0.85 mmol) was dissolved in 10 ml of ethanol, followed by addition of 6 ml of 1 N NaOH and 44 ml of water. The mixture was heated under reflux for 2 hours. After further addition of 60 ml of 0.1 N NaOH to the mixture, heating was continued under reflux for further 2 hours. The reaction mixture was then neutralized with 1 N HCl, the product was extracted with $CHCl_3$, the extract was washed with saturated aqueous sodium hydroxide solution and then with water, the chloroform was removed under reduced pressure, and the syrup thus obtained was allowed to stand overnight in a refrigerator to give the title compound as white crystals (200 mg, m.p. 245-247°C). Further, the filtrate was purified by $C_{18}$ silica gel chromatography (10 g; solvent: 50-90%

acetone-water).

Elemental analysis (%) for $C_{31}H_{31}N_5O_4$, molecular weight 537.618

Calculated : C, 69.26; H, 5.81; N, 13.03

Found : C, 68.80; H, 5.86; N, 12.60

NMR (100MHz, DMSO-$d_6$) $\delta$ ppm: 1.46-2.46-2.46 - (5H, m, $2H_2'$, $2H_5'$, $H_4'$), 3.00-3.38 (4H, m), 3.75 (3H, s, $CH_3$), 3.96-4.16 (1H, m, $H_3'$), 4.68-4.94 (2H, m), 6.38 (1H, bs, NH), 6.80-7.50 (14H, m), 7.77 (1H, s, $H_8$)

Example 12

Synthesis of 9-[(1R, 3R, 4R)-4-hydroxymethyl-3-hydroxyl-cyclopentan-1-yl]isoguanine

The compound obtained in Example 11 (148 mg, 0.27 mmol) was dissolved in 11 ml of 80% acetic acid and the mixture was stirred at 45°C for 6 hours. Then, the solvent was removed under reduced pressure, followed by azeotropic distillation with water. The reaction mixture in the form of an aqueous solution was washed with ether (10 ml x 2). The water was distilled off under reduced pressure and the glass-like substance obtained was suspended in ethanol to give the title compound - [69 mg, m.p. 162-165°C (decomposition)].

Elemental analysis (%) for $C_{11}H_{15}N_5O_3 \bullet 0.5H_2O \bullet 0.1EtOH$, molecular weight 278.887

Calculated: C, 48.24; H, 6.00; N, 25.11

Found : C, 47,92; H, 6.05; N, 24.89

$\lambda_{max}$ (nm): ($H_2O$); 249, 253 (sh), 294 ($H^+$) ; 236, 242 (sh), 283 ($OH^-$); 250, 285

NMR (100MHz,DMSO-$d_6$ + $D_2O$) $\delta$ ppm: 1.10-2.60 - (5H, m, $2H_2'$, $2H_5'$, $H_4'$), 3.40-3.60 (2H, m, $2H_6'$), 4.00-4.20 (1H, m, $H_3'$), 4.60-5.10 (1H, m, $H_1'$), 7.90 - (1H, s, $H_8$)

Example 13

Synthesis of compound of Example 4 - (alternative method)

The compound of Example 8 (420 mg) was dissolved in 10 ml of 6 N sodium hydroxide and the solution was quickly heated and refluxed for 1 hour. After cooling to room temperature, the reaction mixture was neutralized with 1 N hydrochloric acid, purified by HP-20 chromatography (190 ml; solvent: water and 10% ethanol-water) and concentrated to give colorless crystals (174 mg), m.p. 246-248°C.

Reference Example 1

Synthesis of 9-[(1R, 2S, 3R, 4R)-4-monomethoxytrityloxymethyl-2,3-(dimethylmethylene) dioxycyclopentan-1-yl]-hypoxanthine

The C-analog to inosine (10 g 37.6 mmol) was suspended in 380 ml of acetone. To the suspension were added 2,2-dimethoxypropane (23 ml) and p-tosylic acid (9.3 g), and the mixture was stirred at 37°C for 3 hours. After addition of 40 ml of concentrated aqueous ammonia with ice cooling and the subsequent concentration under reduced pressure, the insoluble matter yielded was collected by filtration, purified by $C_8$ silica gel chromatography - (Ø 5.0 x 6.5 cm; solvent: 5% acetone-water) and recrystallized from ethanol to give 8.4 g of a crystalline compound. The filtrate from the recrystallization step was purified by silica gel chromatography (60 g; solvent: $CHCl_3$/MeOH = 25/1 to 5/1) to give a certain amount of the compound as a syrup.

The above crystals (8.4 g, 27 mmol) were suspended in a small amount of pyridine. After azeotropical dehydration under reduced pressure, 150 ml of anhydrous pyridine was added for dissolution of the residue. To the solution was added monomethoxytrityl chloride (9 g) and the mixture was allowed to stand at room temperature for 13 hours. Following addition of 20 ml of methanol, the whole solvent was removed under reduced pressure and the residue was dissolved in 150 ml of $CHCl_3$. The solution was washed with 0.1 M TEAB buffer (100 ml x 2) and then with water (100 ml), dried (anhydrous sodium sulfate) and concentrated by removal of the $CHCl_3$ under reduced pressure, whereupon the title compound precipitated as crystals (9.4 g), m.p. 194-195°C. Similarly, the abovementioned syrup was monomethoxytritylated and the product was combined with the previous recrystallization mother liquor. The combined mixture was dissolved in 70 ml of $CHCl_3$, the solution was added dropwise to 1 liter of n-hexane with stirring, and the resultant precipitate was collected by filtration and dried to give further 10.3 g of the title compound as a powder.

Reference Example 2

Synthesis of 9-[(1R, 2S, 3R, 4R)-4-monomethoxytrityloxymethyl -2,3-(dimethylmethylene)

dioxycyclopentan-1-yl]-(1-methoxymethylhypoxanthine)

The compound obtained in Reference Example 1 (19.1 g, 33 mmol) was dissolved in 360 ml of dioxane-dimethylformamide (DMF) (3:1, v/v), followed by addition of 1.47 g of sodium hydride - (60% in oil) with ice cooling. The mixture was immediately restored to room temperature and stirred for 1 hour. Then, 3.2 ml of methoxymethyl chloride was added with ice cooling and the mixture was stirred for 4 hours with ice cooling. The solvent was removed under reduced pressure and the residue was dissolved in 200 ml of $CHCl_3$. The solution was washed with 0.1 M TEAB buffer (200 ml x 2) and then with water (200 ml), and dried (anhydrous sodium sulfate), and the solvent was removed under reduced pressure. The thus-obtained glass-like substance was roughly purified by silica gel chromatography (100 g; solvent: $CHCl_3$/MeOH = 100/1 to 10/1 and further purified by $C_8$ silica gel chromatography (ø 5.3 x 7.0 cm; solvent: 55-90% acetone-water) to give 16.8 g of a glass-like substance, m.p. 97-102°C.

NMR(90MHz,$CDCl_3$) δ ppm: 1.28 (3H, s, $CH_3$), 1.54 (3H, s, $CH_3$), 2.33-2.60 (3H, m, $H_4'$, $H_5'$), 3.10-3.40 - (2H, m, $H_6'$), 3.43 (3H, s, $CH_2OC\underline{H}_3$), 3.79 (3H, S,

$CH_2OC\underline{H}_3$), 3.79 (3H, S, 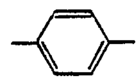

$OCH_3$), 4.47-5.07 (3H, m, $H_1'$, $H_2'$, $H_3'$), 5.46 (2H, s, N-$C\underline{H}_2$-O), 6.79 (1H, s), 6.88 (1H, s), 7.13-7.57 - (12H, m), 7.82 (1H, s), 8.04 (1H, s)

Reference Example 3

Synthesis of 1-[(1R, 2S, 3R, 4R)-4-monomethoxytrityloxymethyl-2,3-(dimethylmethylene) dioxycyclopentan-1-yl]-(4-carbamoyl-5-aminomidazole)

The compound obtained in Reference Example 2 (16.8 g, 26.5 mmol) was dissolved in ethanol (685 ml) and, with heating under reflux, 137 ml of 5 M sodium hydroxide, warmed to about 80°C, was quickly added. Refluxing was continued for 20 minutes and, then, the ethanol was removed under reduced pressure. The product was extracted with 300 ml of $CHCl_3$ and the extract was washed with water (300 ml x 2), 0.1 M TEAB buffer (300 ml) and saturated aqueous sodium chloride solution (300 ml) in that order, and dried (anhydrous sodium sulfate). Rough purification by silica gel chromatography (100 g; solvent: $CHCl_3$/MeOH = 100/1 to 50/1) followed by four repetitions of such silica gel chromatography gave 7.9 g of a colorless glass-like substance, m.p. 107-112°C.

NMR(90MHz,$CDCl_3$)δ ppm: 1.27 (3H, s), 1.58 (3H, s), 1.90-2.67 (3H, m), 3.23-3.40 (2H, m), 3.80 (3H, s), 4.07-4.50 (3H, m), 5.54 (2H, bs, $NH_2$), 6.80 (1H, s), 6.21 (2H, s), 7.20-7.57 (13H, m)

Reference Example 4

Synthesis of 1-[(1R, 2S, 3R, 4R)-4-hydroxymethyl-2,3-(dimethylmethylene) dioxycyclopentan-1-yl]-(4-carbamoyl-5-aminoimidazole) (compound of Reference Example 4-1) and synthesis of 1-[(1R, 2S, 3R, 4R)-4-hydroxymethyl-2,3-dihydroxycyclopentan-1-yl]-(4-carbamoyl-5-aminoimidazole) (compound of Reference Example 4-2)

The compound obtained in Reference Example 3 (7.9 g, 13.9 mmol) was dissolved in 120 ml of 80% acetic acid and the solution was stirred at 40°C for 7 hours. The reaction mixture was concentrated under reduced pressure and subjected to several repetitions of azeotropic distillation with water added, the residue was distributed between water and ether, the aqueous layer was washed with ether and then concentrated under reduced pressure. The concentrate was purified by $C_8$ silica gel chromatography. Fractions eluted with water and 5% acetone-water collected, the solvent was removed under reduced pressure and azeotropic distillation with ethanol was repeated twice to give the compound of Reference Example 4-2 as a crystalline precipitate (0.34 g, m.p. 212-214°C).

Separately, fractions eluted with 15% acetone-water were collected, the solvent was removed under reduced pressure and azeotropic distillation with ethanol was conducted to give the above-mentioned compound of Reference Example 4-1 as crystals weighing 1.85 g, m.p. 169-1/1°C (decomposition). Characteristic data for the compound of Reference Example 4-1:

Elemental analysis (%) for $C_{13}H_{20}N_4O_4 \cdot 0.20H_2O$, molecular weight 299.928

Calculated : C, 52,06; H, 6.86; N, 18.68

Found : C, 52.18; H, 6.60 N, 18.63

NMR (100 MHz,DMSO-$d_6$) δ ppm: 1.26 (3H, s), 1.50 (3H, 3), 1.70-2.40 (3H, m), 3.40-3.60 (2H, m), 4.16-4.82 (4H, m, $H_1'$, $H_2'$, $H_3'$, OH), 5.72 (2H, bs, $NH_2$), 6.65 (2H, bs, $NH_2$), 7.26 (1H, s, $H_2$)

Characteristic data for the compound of Reference Example 4-2:

Elemental analysis (%) for $C_{10}H_{16}N_4O_4$•0.2$C_2H_5OH$•0.5$H_2O$, molecular weight 274.482

Calculated: C, 45.50; H, 6.63; N, 20.42

Found : C, 45.98; H, 6.32; N, 20.07

NMR (100MHz,DMSO-$d_6$) δ ppm: 1.00-2.40 (3H, m, $H_4'$, $H_5'$), 3.38-3.54 (2H, m, $H_6'$), 3.68-4.34 (3H, m, $H_1'$, $H_2'$, $H_3'$), 4.56-5.00 (3H, m, OH), 5.69 (2H, bs, $NH_2$), 6.63 (2H, bs, $NH_2$), 7.22 (1H, s, $H_2$)

Example 14

Synthesis of 1-[(1R, 2S, 3R, 4R)-4-hydroxymethyl-2,3-(dimethylmethylene) dioxycyclopentan-1-yl]-4[4-carbamoyl-5-(N-benzoyl-S-methylisothiocarbamoyl) aminoimidazole]

The compound of Reference Example 4-1 (300 mg, 1 mmol) was suspended in 18 ml of acetone and, with heating under reflux, an acetone solution (5 ml) containing 150 µl of benzoylisothiocyanate was added dropwise over 10 minutes. Refluxing was continued for further 1 hour, the solvent was then removed under reduced pressure, and the residue was dissolved in 5 ml of $CHCl_3$-acetone - (1:1, v/v). Dropwise addition of the solution to 100 ml of n-hexane with stirring gave a precipitate, which was collected by centrifugation and dried to give a light-yellow powder. This powder was dissolved in 10 ml of 0.2 N sodium hydroxide and, following addition of 110 µl of dimethyl sulfate, the mixture was stirred for 1.5 hours. Several drops of acetic acid were added with ice cooling (pH 6-7) and the resultant precipitate was extracted with $CHCl_3$ (10 ml x 2). The extract was washed with water (20 ml x 3), dried (anhydrous sodium sulfate) and purified by silica gel chromatography (11 g; solvent: $CHCl_3$/MeOH -100/1 to 40/1) to give a colorless glass-like substance. This was dissolved in 6 ml of dichloromethane and the solution was added dropwise to 100 ml of n-hexane with stirring. The resultant precipitate was collected by centrifugation and dried. Thus was obtained the title compound as a white powder (320 mg).

Elemental analysis (%) for $C_{22}H_{27}N_5O_5S$•0.5$H_2$), molecular weight 482.561

Calculated : C, 54.76; H, 5.85; N, 14.51

Found : C, 54.75; H, 5.40; N, 14.35

$\lambda_{max}$ (nm): (EtOH); 239, 321 (sh) ($H^+$) ; 247, 305 - (sh) ($OH^-$) ; 264 (sh)

NMR (100MHz,$CDCl_3$) δ ppm: 1.30 (3H, s, $CH_3$), 1.54 (3H, s, $CH_3$), 2.0-2.52 (3H, m, $H_4'$, $H_5'$), 2.58 - (3H, s, S-$CH_3$), 3.75 (2H, d, $H_6'$), 4.48-4.96 (3H, m, $H_1'$, $H_2'$, $H_3'$), 5.85 (1H, bs, NH), 7.58 (1H, s, NH), 7.26-7.54 (4H, m), 7.80-7.94 (2H, m)

Example 15

Synthesis of 9-[(1R, 2S, 3R, 4R)-4-hydroxymethyl-2,3-(dimethylmethylene) dioxycyclopentan-1-yl]guanine

The compound obtained in Example 14 (820 mg, 1.75 mmol) was added to 14 ml of 6 N sodium hydroxide and the mixture was heated quickly and stirred for dissolution thereof. After an hour of continued heating under reflux, the reaction mixture was neutralized with 1 N hydrochloric acid with ice cooling. Rough purification by $C_{18}$ silica gel chromatography (10 g; solvent: water and 20% acetone-water), washing of the aqueous eluate solution with $CHCl_3$, concentration under reduced pressure and crystallization from water gave 410 mg of crystals, m.p. 287-288°C.

Elemental analysis (%) for $C_{14}H_{19}N_5O_4$•0.80$H_2O$, molecular weight 335.747

Calculated : C, 50.08; H, 6.18; N, 20.86

Found : C, 50.28; H, 5.89; N, 20.86 $\lambda_{max}$ (nm): - ($H_2O$); 254, 274, 274 (sh) ($H^+$) ; 256, 281 ($OH^-$); 258 (sh), 270

NMR (100MHz,DMSO-$d_6$+$D_2O$) δ ppm: 1.26 (3H, s, $CH_3$), 1.51 (3H, s, $CH_3$), 1.84-2.40 (3H, m, $H_4'$, $H_5'$), 3.53 (2H, d, $H_6'$), 4.46-5.04 (3H, m, $H_1'$, $H_2'$, $H_3'$), 7.86 (1H, s, $H_8$); (100MHz,DMSO-$d_6$ δ ppm: 3.31 - (1H, bs, OH), 6.63 (2H, s, $NH_2$), 10.85 (1H, bs, NH)

Example 16

Synthesis of 9-[(1R, 2S, 3R, 4R)-4-hydroxymethyl-2,3-dihydroxyl-cyclopentan-1-yl]-guanine

The compound obtained in Example 15 (150 mg, 0.46 mmol) was dissolved in 20 ml of 0.05 N

HCl and the solution was heated at 70°C for 20 minutes.

The reaction mixture was neutralized with 1 N NaOH with ice cooling to give the title compound as crystals (91 mg), m.p. 268-270°C - (decomposition).

$[\alpha]_D^{25}$ = -31.4° (C=0.67, DMF)

Elemental analysis (%) for $C_{11}H_{15}N_5O_4 \bullet 0.7H_2O$, molecular weight 293.882

Calculated : C, 44.96; H, 5.62; N, 23.83

Found : C, 45.19; H, 5.95; N, 23.65

Example 17

Synthesis of 9-[(1R, 3R, 4R)]-4-hydroxymethyl-3-hydroxylcyclopentan-1-yl]hypoxanthine

In 200 ml of toluene was dissolved 12.37 g of 9-[(1R, 3R, 4R)-4-methyl-3,6-(tetraisopropyldisiloxanyl)-dioxycyclopentan-1-yl]-hypoxanthine obtained by the method of Example 3, 10 g of tetrabutylammonium fluoride was added to the solution and then the mixture was stirred at 75°C for 2 hours. The reaction solution was concentrated to dryness, the residue was dissolved in water and adsorbed on the column (30 g) of activated carbon. The substance which was eluted from the column with 1 ℓ of 50% ethanol-water was recrystallized from ether-methanol to give the title compound (2.6 g), m.p. 170°C.

Elemental analysis (%) for $C_{11}H_{14}N_4O_3 \bullet H_2O$

Calculated : C, 49.25; H, 6.01; N, 20.88

Found : C, 49.08; H, 5.86; N, 20.81

Example 18

Tables for oral administration of the antiviral composition according to the present invention are prepared as exemplified below:

Ten(10) mg of 9-[(1R, 3R, 4R)-4-hydroxymethyl-3-hydroxyl-cyclopentan-1-yl]-guanine (the compound of Example 10), 250 mg of lactose, 50 mg of starch and 2 mg of magnesium stearate are mixed in methanol and after removal of methanol with heating is molded into tablets.

Example 19

Ointment for the antiviral composition are prepared as exemplified below:

0.1 g of 9-[(1R, 3R, 4R)-4-hydroxymethyl-3-hydroxylcyclopentan-1-yl]guanine (the compound of Example 10), 40.0 g of vaselinum album, 18.0 g of ethanol, 5.0 g of sorbitan-sesquioleate, 0.5 g of polyoxyethylenelaurylalcohol-ether, 0.1 g of methyl-p-hydroxybenzoate and 36.3 g of purified water are mixed to prepare 0.1% ointments.

Test Example 1

Inhibitory Effect of Antiviral Compounds against Herpes Simplex Virus Type 1, HF Strain

| Compound | $ID_{50}$ [a] (μg/ml) | Cytotoxicity[b] (μg/ml) |
|---|---|---|
| 9-[(1R,3R,4R)-4-hydroxymethyl-3-hydroxyl-cyclopentan-1-yl]-guanine | 0.049 | 12.5 |
| Acyclovir | 3.13 | 800 |
| BUDR | 25 | 100 |
| IUDR | 50 | 200 |
| Ara-A | 12.5 | 100 |
| Ara-C | 0.39 | 0.78 |

Vero cells were infected with 100 $TCID_{50}$ (50% tissue-culture infected doses) of viruses and cultured with various concentrations of the compounds. Antiviral activity ($ID_{50}$; 50% inhibitory dose) was observed three days after the infection, when the control culture without any drug showed 100% cytopathogenic effects. Cytotoxicity was observed with the non-infected control culture at the same time.

a) the concentration required to reduce the viral cytopathogenic effect by 50%

b) the minimum concentration of compounds to cause a visible disruption of normal cell morphology

Test Example 2

Tests of Antiviral Drugs for Antiadenovirus-Activity

| Compound | Adenovirus, Type 3 | |
|---|---|---|
| | $ID_{50}$ (µg/ml) | Cytotoxicity (µg/ml) |
| 9-[(1R,3R,4R)-4-hydroxymethyl-3-hydroxyl-cyclopentan-1-yl]-guanosine | 0.39 | 3.13 |
| Acyclovir | 200 | 400 |

FL cells were infected with 100 $TCID_{50}$ (50% tissueculture infected doses) of viruses and cultured with various concentrations of the compouns. Antiviral activity ($ID_{50}$; 50% inhibitory dose) was observed eight days after the infection, when the control culture without any drug showed 100% cytopathogenic effects. Cytotoxicity was observed with the non-infected control culture at the same time.

## Claims

1. A compound of the formula

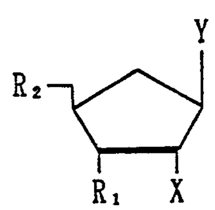

wherein $R_1$ and $R_2$ each is a hydroxyl group, which may optionally be protected, X is a hydrogen atom or a hydroxyl group, which may optionally be protected, and Y is guanin-9-yl, isoguanin-9-yl or hypoxanthin-9-yl, provided that when X is a hydroxyl group, which may optionally be protected, Y is other than hypoxanthin-9-yl.

2. The compound according to claim 1, wherein $R_1$ and $R_2$ each is a hydroxyl group, X is a hydrogen atom and Y is guanin-9-yl.

3. The compound according to claim 1, wherein $R_1$ and $R_2$ is a hydroxyl group, X is a hydrogen atom and Y is isoguanin-9-yl.

4. The compound according to claim 1, wherein $R_1$ and $R_2$ each is a hydroxyl group, X is a hydrogen atom and Y is hypoxanthin-9-yl.

5. The compound according to claim 1, wherein $R_1$, $R_2$ and X each is a hydroxyl group and Y is guanin-9-yl.

6. A method of producing compounds of the formula

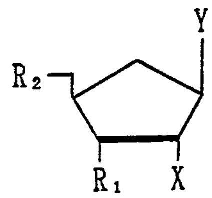

wherein $R_1$ and $R_2$ is a hydroxyl group, which may optionally be protected, X is a hydrogen atom or a hydroxyl group, which may optionally be protected, and Y is guanin-9-yl, isoguanin-9-yl or hypoxanthin-9-yl, provided that when X is a hydroxyl group, which may optionally be protected, Y is other than hypoxanthin-9-yl, which comprises subjecting a compound of the formula

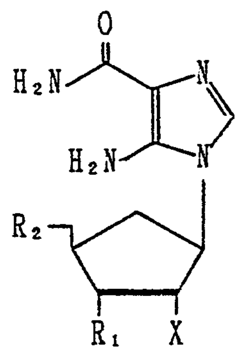

wherein $R_1$, $R_2$ and X are as defined above, to purine ring formation reaction.

7. A compound of the formula

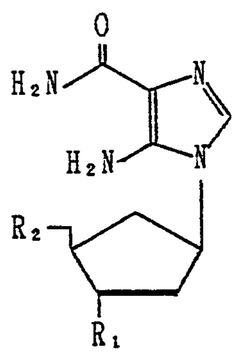

wherein $R_1$ and $R_2$ each is a hydroxyl group, which may optionally be protected.

8. An antiviral composition containing a compound of the formula

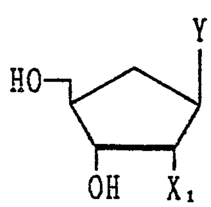

wherein $X_1$ is a hydrogen atom or a hydroxyl group, and Y is guanin-9-yl, isoguanin-9-yl or hypoxanthin-9-yl, provided that when $X_1$ is a hydroxyl group, Y is other than hypoxanthin-9-yl.

9. The composition according to claim 8, wherein $X_1$ is a hydrogen atom and Y is guanin-9-yl.

VON KREISLER SCHÖNWALD EISHOLD FUES
VON KREISLER KELLER SELTING WERNER

PATENTANWÄLTE

Dr.-Ing. von Kreisler † 1973
Dr.-Ing. K. W. Eishold † 1981
Dr.-Ing. K. Schönwald
Dr. J. F. Fues
Dipl.-Chem. Alek von Kreisler
Dipl.-Chem. Carola Keller
Dipl.-Ing. G. Selting
Dr. H.-K. Werner

DEICHMANNHAUS AM HAUPTBAHNHOF
D-5000 KÖLN 1

European Patent Office
Rijswijk
Niederlande

AvK/IM January 19,1987

86 114 466.5 ✓
Takeda Chemical Industries, Ltd.

---

Applicant has noticed that the chemical names of the respective compounds produced in Examples 3 - 12 and 17 - 19 are not described correctly.

"...(1R,3R,4R)..." in the chemical names should be corrected to "...(1R,3S,4R...)".

Enclosed are in triplicate new pages 9, 10, 12 to 17, and 25 to 27 in which these amendments have been made in handwriting.

It is respectfully requested to exchange the enclosed pages against the originally filed pages.

Patent Attorney

(Alek von Kreisler)

Encls.

Telefon: (02 21) 13 10 41 · Telex: 888 2307 dopa d · Telegramm: Dompatent Köln